# EUROPEAN PATENT SPECIFICATION

(11) **EP 2 456 324 B1**
(45) Date of publication and mention of the grant of the patent: **04.09.2013**
(21) Application number: 10755249.9
(22) Date of filing: 21.07.2010
(51) Int. Cl.: A23K 1/18, A01K 67/033

(54) **SYSTEM AND METHOD OF FEEDING BENEFICIAL INSECTS**
SYSTEM UND VERFAHREN ZUR FÜTTERUNG VON NÜTZLICHEN INSEKTEN
SYSTÈME ET PROCÉDÉ POUR NOURRIR DES INSECTES BÉNÉFIQUES

(30) Priority: 23.07.2009 US 227811 P
(43) Date of publication of application: 30.05.2012
(73) Proprietor: BIO-FLY AN AGRICULTURAL COOPERATION SOCIETY LTD, Kibbutz Sde Eliyahu 10810 (IL)
(72) Inventor: SHOUSTER-DAGAN, Inbar, 10810 Kibbutz Sde Eliyahu (IL); LELLOUCHE, Ayala, 10810 Kibbutz Sde Eliyahu (IL); FREUND, Myriam, 10810 Kibbutz Sde Eliyahu (IL); ZUNZ, Hillel, 10810 Kibbutz Sde Eliyahu (IL); ROTEM, Moshe, 10810 Kibbutz Sde Eliyahu (IL); LEVI, Ofir, 10810 Kibbutz Sde Eliyahu (IL); STEINBERG, Shimon, 10810 Kibbutz Sde Eliyahu (IL)
(74) Representative: Lecomte & Partners
(86) International application number: PCT/IL2010/000578
(87) International publication number: WO 2011/010308

(56) References cited:
- EP-A2- 2 248 417
- WO-A1-81/00037
- US-A- 5 799 607
- US-B1- 7 354 611

## Description

### BACKGROUND OF THE INVENTION

### 1. Field of the Invention

The present invention relates generally to the feeding and diet of insects and particularly to methods of producing a diet for insect biological control agents derived from insect eggs.

### 2. Background Art

The use of insects as Biological Control Agents (BCAs) in agriculture is an ever-expanding market, worth approximately US$350 million annually, not including bumblebees, and microbials. BCAs such as the minute pirate bug (e.g. *Orius laevigatus, O. insidiosus*) are artificially reared and then applied on crops. The minute pirate bug is an important predator of thrips, phytophagous mites (eggs and mobile stages), insect eggs and a number of soft bodied insect pests. In greenhouse vegetables (chiefly sweet pepper, egg plant, melon and others) its role as a predator of Western flower thrips, sweet potato whitefly and spider mites is particularly significant and economically important to pest managers and integrated pest management programs. Minute pirate bugs can be found naturally in important open field crops such as com, soybean, alfalfa, and cotton. The minute pirate bug possesses highly efficient searching behavior and is a voracious feeder, each individual being capable of consuming 30 or more spider mites and Western flower thrips a day. While it preys on insects that are pests to the crops, the damage it causes itself to the crops is negligible, thus commercially reared minute pirate bugs are widely used in agriculture in open fields and greenhouses as a form of biological control.

*Orius* bugs are available commercially from insectaries and are shipped as adults in a carrier such as bran, rice hulls, or vermiculite, along with a food source. The carrier can be shaken onto plants and the bugs will readily disperse and locate prey. Several patents in the field, for example, CN21192036Y and CN101331868 (A) relate generally to devices and methods for housing, feeding, breeding and rearing minute pirate bugs.

Other BCAs include Bigeyed bugs that feed on insect eggs and many other pests such as flea beetles, caterpillars and spider mites. Ladybeetles feed on aphids, mealybugs, spider mites and other soft-bodied arthropods. The green lacewing feeds on soft-bodied arthropods including aphids, thrips, mealybugs, scales, caterpillars and mites. Predatory mites such as *Phytoseiulus persimilis,* that feed on spider mites, are also extensively used in agriculture. All these BCAs can be reared commercially to be used for biological control of different pests on various crops.

Patents exist for the general rearing, feeding, breeding and transporting of these BCAs, such as US5784991 that describes a method for rearing or transporting entomophagous insects by providing a shelter made of foam. CN1631127A describes a system of artificial rearing of lacewings and ladybugs by providing them with all the steps necessary to maintain their lifecycle. US6506597, US5945271 and US6235528 describe an artificial diet for entomophagous insects comprising cooked avian egg and a plurality of other constituents however this diet is not based on the natural diet of the BCAs.

EP0827375B1 provides a method for breeding and packaging auxiliary organisms such as lacewings and predacious plant bugs for biocontrolling plant pests. According to the method, breeding boxes are filled with a substrate such as popcorn coated with an adhesive material so that a specific feed dose required for breeding the BCA can be maintained, the food consisting of the eggs of the flour moth, *Ephestia kuehniella.* US7354611 describes a new protein supplement for insect rearing of that contains extracts of insect eggs or cultured insect egg embryonic cell lines from insects such as *Plodia interpunctella* and *Ephestia kuehniella* to increase the fecundity of insects mass-reared for biocontrol such as the minute pirate bug. US4765274 also describes a method of mass producing an insect egg-parasitoid such as *Trichogramma maidis* by providing host eggs of E. *kuehniella.* However the use of *E. kuehniella* as a diet for beneficial insects (especially predators) is expensive and significantly increases the cost of mass rearing them for biological control purposes.

The Mediterranean fruit fly, also known as the medfly, is one of the major pests in agriculture, able to cause damage to a wide range of fruit crops. The female medfly lays its eggs under the skins of fruit. They hatch after about three days, depending on temperature. The larvae develop inside the fruit causing severe damage. An important non-chemical and environmentally-benign means of controlling the medfly is the Sterile Insect Technique (SIT) where male pupae of the medfly are irradiated, giving rise to sterile adults. Sterile males are mass-released into the field. Once they mate with wild females the latter lay eggs that will fail to hatch thus decimating the future population. In this way the medfly or other insect pests can be controlled and even eradicated in the case of some species, in a species-specific manner without the use of pesticides. The by-product of medfly mass production for use in SIT programs are medfly eggs which may be processed for other purposes, such as the production of an alternative diet for minute pirate bugs and other beneficial insects.

Thus it is a long felt need in the field of plant protection in agriculture to provide a cost effective and nutritionally-suitable alternative or factitious diet for BCAs mass-reared for commercial use. The present invention solves many of these problems by introducing a new method of processing insect eggs, especially those of the fruit fly, which may be derived as an SIT by-product or produced especially for the purpose, suitable for feeding beneficial insects.

### SUMMARY OF THE INVENTION

The present invention relates to the field of insect diets and more particularly to an insect diet suitable for rearing insect biological control agents, essentially derived from medfly insect eggs produced especially for the purpose or as a by-product of insect control such as sterile insect technique. More specifically the present invention discloses a method of preparing such an insect diet.

It is one object of the present invention to disclose a system of rearing insect biological control agents (BCAs) that comprises nutrients provided to the BCAs, the nutrients being essentially derived from Processed Insect Eggs (PIE). It is also in the scope of the present invention to disclose a system of rearing BCAs in which the processed insect eggs are derived from medfly eggs as a by-product of medfly mass-production for use in sterile insect technique. It is also in the scope of the present invention that the insect eggs are derived from medfly or other insects specifically reared for the purpose.

It is also in the scope of the present invention to disclose a system of rearing BCAs in which the insect eggs of the alternative insect diet are derived from any Tephritid species, particularly the olive fly and the medfly.

It is also in the scope of the present invention to disclose a system of rearing insect BCA's such that the nutrients are provided by PIE in combination with other nutrients.

It is also in the scope of the present invention to disclose a system of rearing insect BCA's such that the nutrients are provided by PIE in combination with insect BCA nutrients such as *Artemia* eggs and *Ephestia kuehniella* eggs. It is also a preferred embodiment to disclose a system of rearing insect BCA's in which the other nutrients are provided by at least one other different insect species eggs that have undergone the PIE processing system.

In another aspect of the present invention a mixture of PIE and at least one other insect diet is disclosed, such that at least one other insect diet comprises between about 5% and about 50% of the diet supplied to the BCA.

In another aspect of the present invention a system is disclosed that is useful for providing nutrients to insect BCAs, wherein the BCAs are selected from the order *Hemiptera.* It is also in the scope of the present invention to disclose a system useful for providing nutrients to insect BCA's selected from the family *Miridae.* It is also in the scope of the present invention to disclose a system useful for providing nutrients to insect BCA's selected from the genus *Nesidiocoris* comprising Mirid bugs *(Nesidiocoris tenuis)* and *Nesidiocoris spp.,* or any combination thereof. It is also in the scope of the present invention to disclose a system useful for providing nutrients to insect BCA's selected from the family *Anthocoridae.* It is also in the scope of the present invention to disclose a system useful for providing nutrients to insect BCA's selected from the genus *Orius,* comprising minute pirate bugs *Orius laevigatus, Orius insidiosus, Orius spp.,* or any combination thereof. It is also in the scope of the present invention to disclose a system useful for providing nutrients to insect BCA's selected from the family *Lygaeidae.* It is also in the scope of the present invention to disclose a system useful for providing nutrients to insect BCA's selected from the genus *Geocoris* comprising big-eyed bugs, *Geocoris spp.,* or any combination thereof.

It is also a preferred embodiment of the present invention to disclose a system useful for providing nutrients to insect BCAs selected from the order *Coleoptera.* It is also in the scope of the present invention to disclose a system useful for providing nutrients to insect BCA's selected from the family *Coccinellidae.* It is also in the scope of the present invention to disclose a system useful for providing nutrients to insect BCA's selected from the genus *Cryptolaemus,* comprising the mealybug predator, the lady beetle (*Cryptolaemus montrouzieri), Cryptolaemus spp.,* or any combination thereof.

It is also a preferred embodiment of the present invention to disclose a system useful for providing nutrients to insect BCAs selected from the order *Neuroptera.* It is also in the scope of the present invention to disclose a system useful for providing nutrients to insect BCA's selected from the family *Chrysopidae.* It is also in the scope of the present invention to disclose a system useful for providing nutrients to insect BCA's selected from the genus *Chrysoperla* comprising the green lacewing, *Chrysoperla spp.,* or any combination thereof.

It is also a preferred embodiment of the present invention to disclose a system useful for providing nutrients to non-insect BCAs of the *Arachnida* class selected from the order *Mesostigmata* of predatory mites. It is also in the scope of the present invention to disclose a system useful for providing nutrients to non-insect BCAs of the *Arachnida* class selected from the family *Phytoseiidae.*

It is also in the scope of the present invention to disclose a system useful for providing nutrients to non-insect BCAs of the *Arachnida* class selected from the genus *Amblyseius;* comprising the predatory mites *Amblyseius swirskii* and *Amblyseius spp.,* or any combination thereof.

It is also in the scope of the present invention to disclose a system useful for providing nutrients to non-insect BCAs of the *Arachnida* class selected from the genus *Phytoseiulus* comprising the predatory mites *Phytoseiulus persimilis, Phytoseiulus longipes* and *Phytoseiulus spp.,* or any combination thereof.

It is also in the scope of the present invention to disclose a system useful for providing nutrients to non-insect BCAs of the *Arachnida* class selected from the genus *Euseius* comprising the predatory mites *Euseius scutalis* and *Euseius spp.,* or any combination thereof

It is one object of the present invention to disclose a PIE produced BCA, wherein the gut contents of the BCA's raised on PIE are substantially different to those of BCA's raised on other BCA diet.

According to another preferred embodiment of the present invention a factitious diet composition comprising PIE is disclosed; the PIE having been dehydrated, mixed with anti-caking agent and stored until use under appropriate conditions of below 0°C. In this embodiment the PIE is suitable for providing nutrients for another different biological control agent and furthermore the PIE are derived from an egg supply comprising egg by-products of insect control programs, for example sterile insect technique in medfly.

In another aspect of the present invention a method of dehydrating insect eggs useful for providing a factitious insect diet is disclosed, comprising the steps of; collecting and storing eggs under aqueous conditions, determining the volume of eggs collected, separating out a solid phase containing the insect eggs, combining the separated insect eggs with a granular desiccant, mixing the desiccant and the eggs such that an intermediate product is provided containing the insect eggs in a non-sticky, non-aggregated formulation.

It is also in the scope of the present invention to disclose a method of providing an intermediate product of insect eggs, comprising the steps of; storing collected eggs under aqueous conditions in a refrigerator, determining the volume of eggs by transferring the eggs to a measuring container and removing excess water, dewatering by centrifugation sufficient for separating out a solid phase containing the insect eggs from the aqueous phase, combining separated insect eggs with a granular desiccant, for example fresh rice, at a rate of approximately 2.5 times the known volume of insect eggs, mixing the desiccant with the insect eggs in a mechanical mixer, with additional manual force for between about 15 to 20 minutes, such that an intermediate product is provided containing the insect eggs in a non-sticky, non-aggregated formulation.

In a preferred embodiment of the present invention a method of separating desiccated insect eggs from an intermediate product is disclosed, comprising the steps of; placing the intermediate product in a filtration machine, collecting the granular desiccant in one container, collecting the dehydrated insect eggs in another different container, such that dehydrated insect eggs are provided for the production of a factitious insect diet.

In another aspect of the present invention a method of preparing PIE from desiccated insect eggs is disclosed comprising the steps of; determining the weight of desiccated insect eggs, coating the insect eggs with an anti-caking agent, mixing the anti-caking agent with the insect eggs to provide a uniform mixture, such that the mixture is useful for providing a factitious insect diet for insect BCAs.

It is also in the scope of the present invention to disclose a method of preparing PIE comprising the steps of; weighing the desiccated insect eggs, coating the insect eggs with an anti-caking agent, more particularly rice flour, at a rate of between about 5% to 8% of the desiccated eggs' weight, mixing the rice flour with the desiccated insect eggs, to provide a uniform mixture, such that the mixture is useful for providing a factitious insect diet for insect BCAs.

In another preferred embodiment of the present invention a method of storing PIE is disclosed, comprising the steps of bagging the PIE into sachets, welding the sachets to seal contents, quick freezing the sachets and contents in a Individually Quick Frozen (IQF) freezer, transferring the sachets to a standard freezer, such that the packaged and frozen PIE provided maintains its nutritional attributes and freshness for a period of not less than one year.

It is yet another object of the present invention to disclose a method of preparing and storing PIE, in all its preparation, desiccation, mixing and storage stages, such that all stages are carried out in a dehumidified environment of relative humidity between about 20% to about 40%.

In another aspect of the present invention a method of storing the PIE in a sachet is disclosed, in which the sachet comprises a high oxygen barrier film useful for maintaining the attributes and freshness of the PIE for a period of not less than one year. In yet another aspect of the present invention, a method of freezing the PIE is disclosed in which IQF freezing is useful for maintaining the attributes and freshness of the PIE for a period of not less than one year. In another aspect of the present invention, a method is disclosed of preparing PIE suitable for distribution as a factitious insect diet for commercially reared BCAs.

In a preferred embodiment of the present invention a covered or packaged factitious insect diet is disclosed in covered or packaged form, comprising insect eggs and an anti-caking agent in a sachet, in which the covered or packaged factitious insect diet is effective for the commercial rearing of a BCA.

### BRIEF DESCRIPTION OF THE DRAWINGS

Figure 1 is a flow chart illustrating the process of desiccating insect eggs in a first stage of preparation of a diet for beneficial insects; and
Figure 2 is a flow chart illustrating the process of adding anti-caking products to the insect eggs and processing them in a second stage of preparation of a diet for beneficial insects; and
Figure 3 is a flow chart illustrating the storage process in a third and final stage of preparation of a diet for beneficial insects; and
Figures 4A and 4B are graphs illustrating the number of *Orius laevigatus* individuals collected (A) and the percent mortality (B) in a given production unit under different feeding regimes; and
Figure 5 is a graph illustrating the percentage of *O. laevigatus* adults in each production unit under different feeding regimes; and
Figures 6A and 6B are graphs illustrating the fecundity (=egg laying capacity) of *O. laevigatus* in different production units under different feeding regimes shown as the number of eggs per female (A) and the amount of offspring produced (B) in each production unit; and
Figure 7 is a graph illustrating the number of offspring produced per production unit under different feeding regimes; and
Figure 8 is a graph illustrating the amount of individual *O. laevigatus* bugs collected from different feeding regimens of 6 months long term storage; and
Figures 9A and B are graphs illustrating the no. of individual *O. laevigatus* bugs collected (A) and the percentage of adults collected (B) in mixed *Ephestia kuehniella* and medfly PIE diets and 8 month long term medfly PIE storage; and
Figure 10 is a pictorial illustration of *E. kuehniella* eggs before and after undergoing processing system and method to become PIE; and
Figures 11A and B are graphs illustrating the number of adult *O. laevigatus* bugs collected (A) and the females' fecundity of *O. laevigatus* feeding upon processed *E. kuehniella* eggs (B).

### DETAILED DESCRIPTION OF THE PREFERRED EMBODIMENTS

The term Processed Insect Eggs (PIE) herein refers to insect eggs that have undergone, in a non limiting manner, any or all or some of the following steps, subsequent to harvesting, such as desiccation or drying, dewatering, anti-caking, various methods of freezing, cooling, packaging, thickening, liquefying, separating, humidifying, rehydrating, mixing, thermally adjusting, warming, centrifuging, addition of any component or composition whatsoever at any stage. The steps may be administered in parallel, in series, contemporaneously or simultaneously.

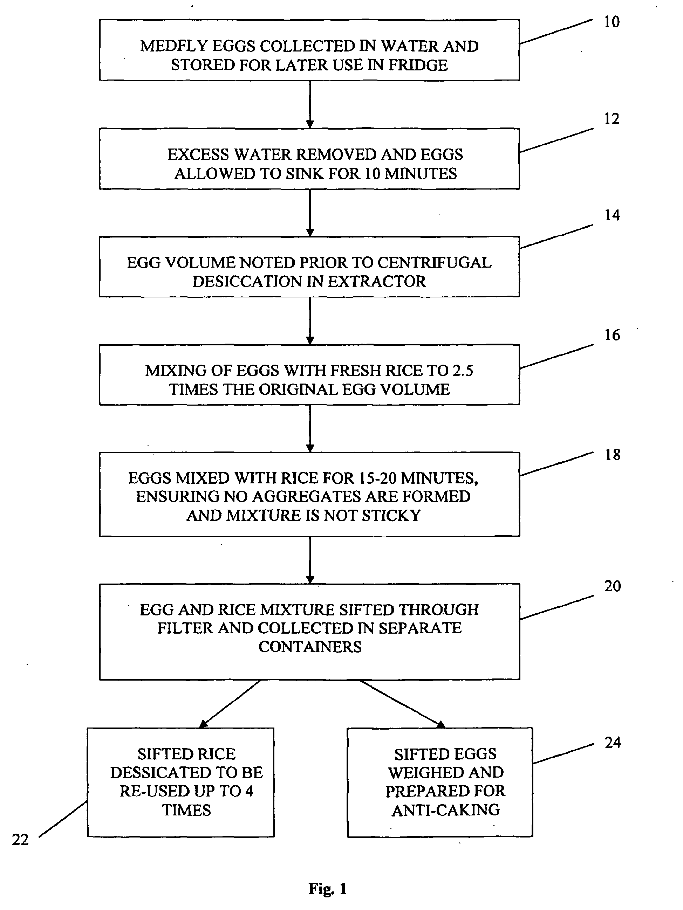

Figure 1 illustrates a process of desiccating insect eggs as a first stage in the preparation of a diet for BCAs such as *Orius laevigatus* according to one embodiment of the present invention. In step 10 Medfly eggs or other suitable eggs are collected and stored under water in a refrigerator. In this embodiment the eggs may be a by-product of sterile insect technique or be specially prepared for the purpose of providing a factitious insect diet. In step 12, excess water is drained from the Medfly eggs and the eggs are allowed to settle at the bottom of a measuring jug for about 10 minutes. Once all the eggs have settled, the volume of eggs is ascertained in step 14 for future reference and the eggs are desiccated in an extractor by centrifugation for 7 seconds while being kept in a fine organdy cloth bag that enables the water to be drained out whereas the eggs stay inside.

In step 16, the insect eggs are further desiccated by mixing the insect eggs with fresh rice in the amount of 2.5 times the original volume of insect eggs. The insect eggs are then mixed together with the rice in step 18 for 15-20 minutes. In this step it is necessary to ensure that aggregates are not formed in the mixture by setting the mixer to its optimal operation. Care should be taken in this step that the mixture does not become sticky.

In step 20, the egg and rice mixture is sifted and separated for example by a filtration machine, or by gusts of air that separate particles according to weight, or indeed by any other method that is capable of separating the components of the mixture. In this step the egg and rice mixture may be passed through the filtration machine or other separation method more than once to ensure complete separation of the dehydrated insect eggs from the rice. The insect eggs and rice are collected into separate containers such that in step 22 the sifted rice is desiccated and may be reused up to four times for the purpose of dehydrating the insect eggs. The sifted insect eggs are collected in step 24 into a container and weighed in preparation for adding the anti-caking agent.

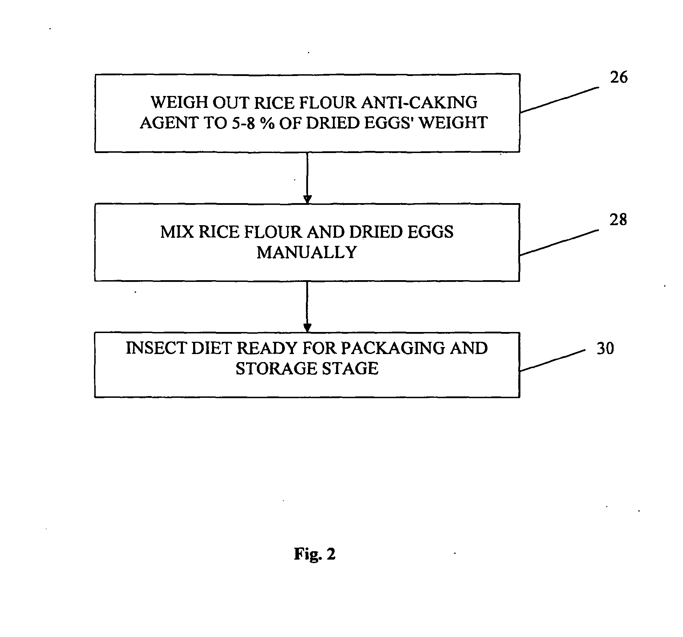

Figure 2 illustrates the process of adding anti-caking products to the insect eggs in a second stage of processing them for distribution as a diet for BCAs. In step 26 the desiccated and weighed insect eggs are mixed with anti-caking rice flour at a rate of 5%-8% by weight of the dried insect eggs. Care should be taken at this step to minimize processing time to prevent crystallization and solidification of the eggs. The rice flour and insect eggs are mixed thoroughly in step 28 manually or by transferring them from container to container or alternatively by mechanical mixing by a machine, to complete the preparation of the insect diet. In step 30, the prepared insect diet is ready to be weighed out into the appropriate portions and packaged.

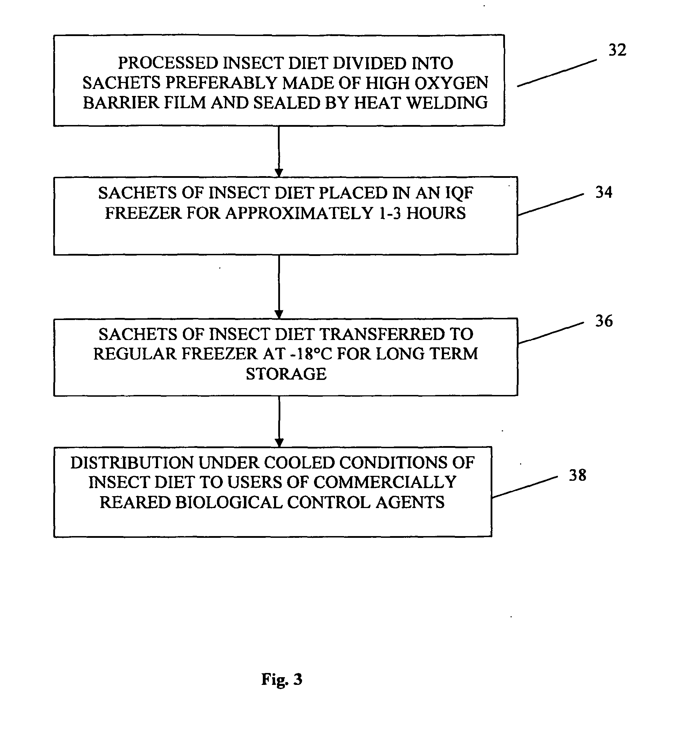

Figure 3 illustrates the third and final stage of preparation of the insect diet in which in step 32, the processed insect diet is divided into sachets made of a high oxygen barrier film. In this step the insect diet may be divided into sachets of equal weight, for example each sachet containing 250g of prepared diet. In a preferred embodiment the high oxygen barrier film is important in maintaining the freshness of the insect diet enabling use of the diet up to one year after preparation. The sachets are then sealed by heat welding. In another preferred embodiment, in step 34 the packaged insect diet is placed in a quick freeze, a process named Individually Quick Frozen (IQF), for 1-3 hours for example, making sure that the sachets are well separated to ensure thorough and even freezing of the contents. The rapid freezing of the insect diet further maintains the freshness of the insect diet and in combination with the high oxygen barrier film sachet, maintains the high quality of the insect diet for at least one year. In step 36 the sachets of the insect diet are stored in a regular freezer, at for example -18°C, for long-term storage. In step 38 the insect diet is distributed under cooled conditions to users that commercially mass rear BCAs.

It is yet another embodiment of the present invention that a dehumidifying system is provided at all the processing and desiccation stages of the PIE preparation system. Thus for example in this embodiment, a supply of dry wind is provided at the mixing stage of the preparation. In addition or alternatively, a dehumidifier is provided at all the stages to provide a level of about 20-40% relative humidity.

It is yet another embodiment of the present invention that the PIE of the present invention comprises at least one type of insect egg that has undergone the aforementioned processing steps, either alone or in combination with other insect egg species that have undergone the same processing steps. Also in this embodiment the PIE is in combination with insect eggs that are unprocessed or have undergone different preparations, for example *Ephestia kuehniella* eggs. Also in this embodiment the PIE is combined with eggs derived from other species, for example the eggs of crustaceans such as the brine shrimp- *Artemia.*

It is yet another preferred embodiment of the present invention that the medfly PIE diet and other PIE diets are adaptable to use with a variety of insect BCA species. In this embodiment the PIE can be used to feed insect BCAs selected from but not limited to the group of insect orders including; *Hemiptera, Neuroptera* and *Coleoptera.* Examples of *Hemiptera* are insects belonging to the *Miridae* family (e.g. the mirid bug - *Nesidiocoris tenius),* to the *Anthocoridae* family (e.g the minute pirate bug - *Orius laevigatus*) and to the *Lygaeidae* family (e.g. big-eyed bugs- *Geocortis spp*). Insects belonging to the *Coleoptera* order include those belonging to the *Coccinellidae* family.(e.g. the mealybug destroyer also known as the mealybug ladybeetle- *Cryptolaemus montrouszieri.* Other BCA species normally fed on a diet of *E. kuehniella* eggs such as the *Chrysopidae* family of the *Neuroptera* order (e.g. the lacewing- *Chrysoperla spp.*) are also included in this embodiment.

In another preferred embodiment of the present invention, the PIE diet is adaptable for rearing, systems of non-insect BCA's. In this embodiment PIE can be used to feed non-insect BCA's from the *Arachnida* class. In this embodiment the PIE diet is useful for feeding predatory mites from the order of *Mesostigmata.* Examples of *Mesostigmata* are predatory mites belonging to the *Phyloseiidae* family, such as those in the genus *Amblyseius* (e.g. *Amblyseius swirskii* and *Amblyseius spp.),* predatory mites belonging to the genus *Phytoseiulus* (e.g. the *Phytoseiulus persimilis, Phytoseiulus longipes* and *Phytoseiulus spp.)* and predatory mites belonging to the genus *Euseius* (e.g. *Euseius scutalis* and *Euseius spp*.).

In another preferred embodiment of the present invention an assessment of the gut contents of the BCA's characterizes those fed on the novel PIE diet of the present invention. In this embodiment a chemical breakdown of the gut contents of a BCA fed on PIE is significantly different to that of a BCA fed on other type of BCA diet.

The following examples are intended to further illustrate the invention and are not intended to limit the scope of the invention. A model system was used wherein the beneficial insect *Orius laevigatus* (minute pirate bug) was supplied with eggs of *Ephestia kuhniella* in a control diet and compared to the new diet. In other examples, the medfly PIE diet or a mixed diet was supplied to *O. laevigatus* and to other BCA insect species.

### EXAMPLE 1

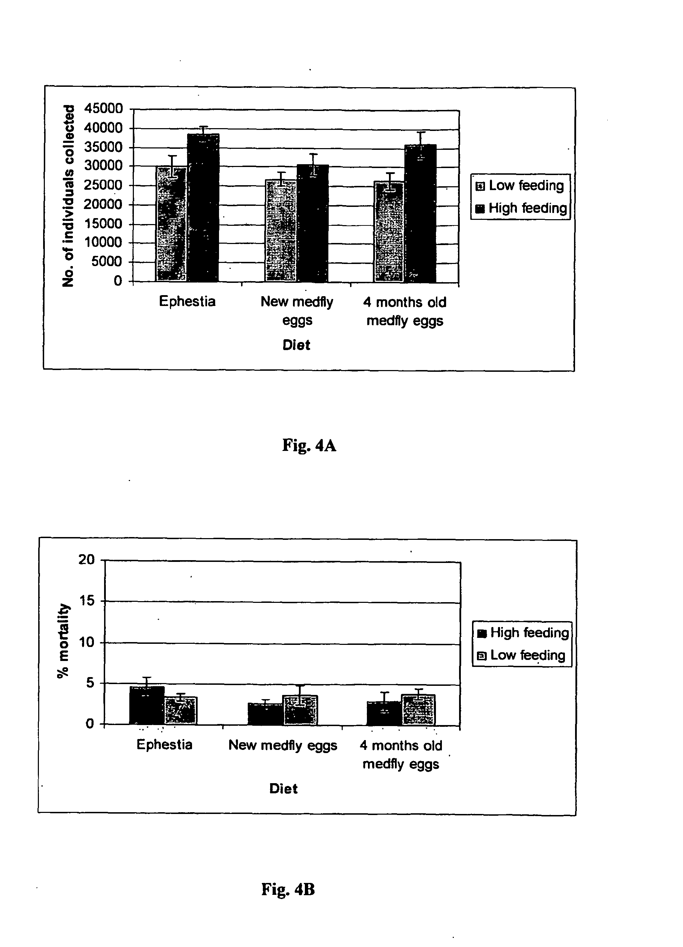

In a bifactorial experiment the amount of food presented (high/low) and the food type (new *E. kuhniella* eggs/new medfly eggs/4 months old medfly eggs) were compared by feeding young bugs, with each regime being replicated 3 times. Each production unit of 35000 *O. laevigatus* nymphs was fed with one of the above diets. At the end of nymphal development the following parameters were assessed: total number of bugs, % adults and % mortality. Figure 3A, shows the amount of individual *O. laevigatus* bugs harvested from different feeding regimes, illustrating that in all types of diet the amount of bugs produced decreases when fed relatively low amounts of food, but was essentially similar between the different kinds of food. Figure 3B illustrates that the percentage mortality is similar in all the different regimes. Figure 4 illustrates that the percentage of adults in the population was lower in production units fed low amounts of food compared to the higher amount, particularly so in the case of production units fed low quantities of 4 months old medfly eggs compared to low quantities of new *E. kuhniella* eggs. Furthermore, the effect of juvenile diet on storage of *O. laevigatus* as adults was tested by storing 90000 bugs for 2 weeks and was found to have no influence.

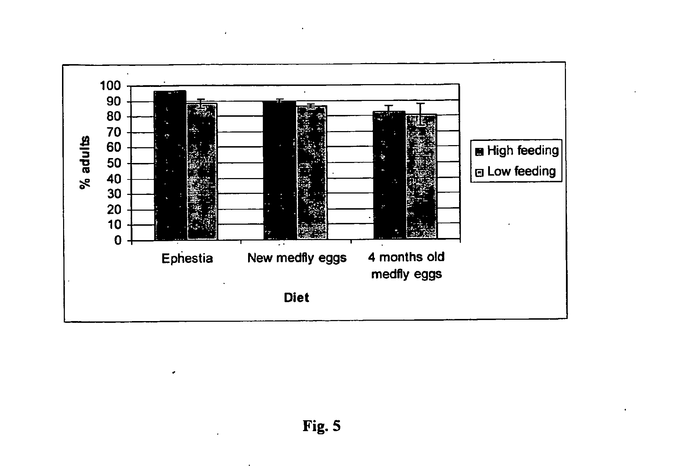

To test for the effect of juvenile diet on the bugs' fecundity, 20 couples of adult bugs obtained from each feeding regime were used for assessing fecundity in individual arenas. Additionally, one industrial egg-laying unit was established from each feeding regime and the total amount of offspring produced was assessed. No significant differences were found between the different regimes in the amount of eggs deposited by a single female (Figure 5A), although females that were reared on medfly eggs laid slightly more eggs than females reared on E. *kuhniella* eggs. In the industrial egg-laying units (Figure 5B) offspring production was similar between the different regimes regardless of the juvenile diet used.

### EXAMPLE 2

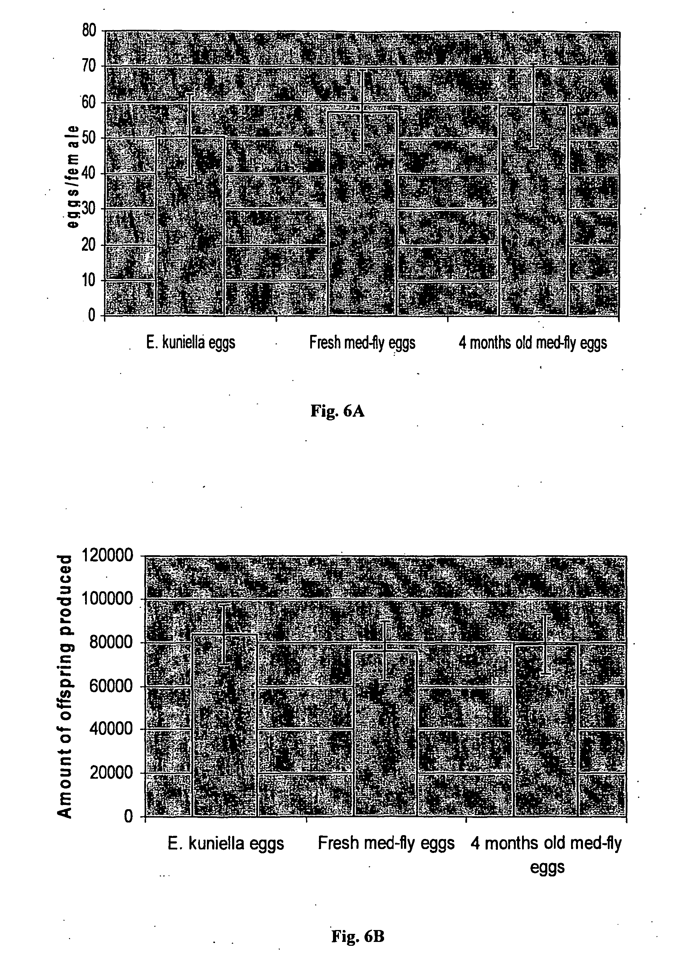

In a second test, juvenile *O. laevigatus* were reared with either medfly or *E. kuhniella* eggs. Upon reaching adulthood industrial egg-laying units were established in which the adult bugs received the same diets as they had as juveniles. It was found that industrial production units produced similar amounts of offspring regardless of juvenile and adult diet (Figure 6).

The diet of the invention produced similar quantities of adults, with similar rates of mortality and fecundity compared to the control *E. kuhniella* diet, commonly used in commercial mass rearing of BCAs.

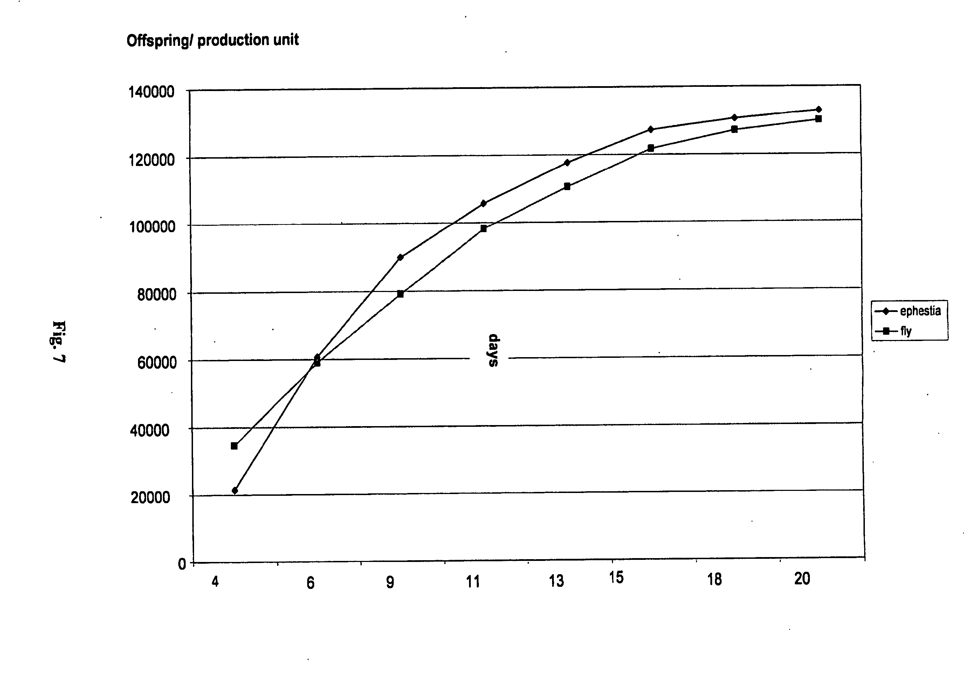

Although the medfly eggs diet gave similar results to *E. kuhniella* eggs diet with respect to number of *O. laevigatus* adults produced, their mortality rate and fecundity (Figure 7), the medfly eggs diet is 35-50% cheaper than a diet based on *E. kuhniella* eggs. Furthermore, the storability of the medfly egg diet is double than that of *E. kuhniella* egg diet.

### EXAMPLE 3

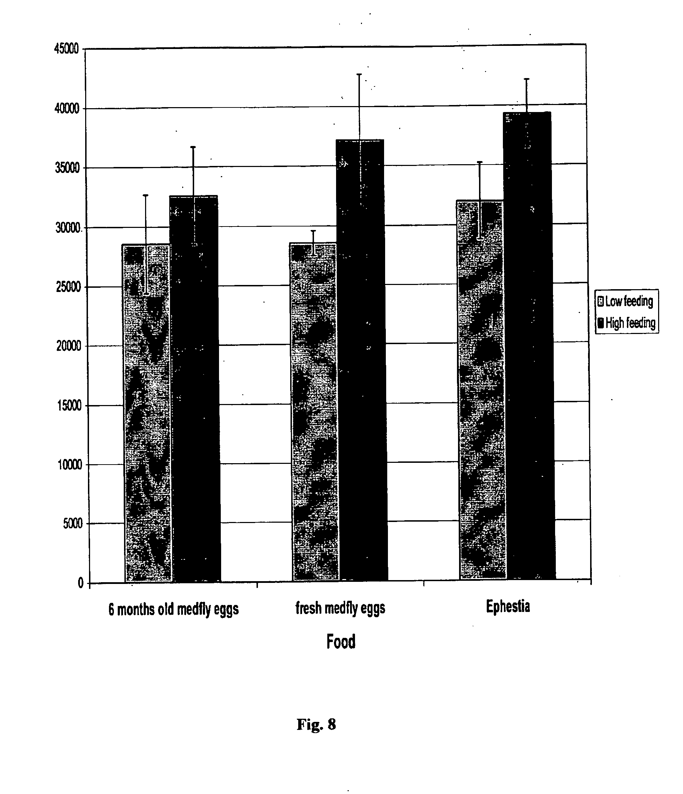

In a further test, the results of long-term storage on the PIE was assessed in a bifactorial experiment. As in Example 1, fresh *E. kuhniella* and fresh medfly PIE eggs were compared in high and low amounts with medfly PIE that had been stored for 6 months. In each treatment approximately 38000 *O. laevigatus* nymphs were fed and each treatment was replicated 5 times. At the end of the experiment the number of bugs harvested was assessed. Figure 8A shows that the number of bugs collected was significantly less when fed a low amount of food but that there was no significant difference between the different diets themselves. Thus long-term storage of the PIE for 6 months does not alter its effectiveness as an insect diet for *O. laevigatus.*

### EXAMPLE 4

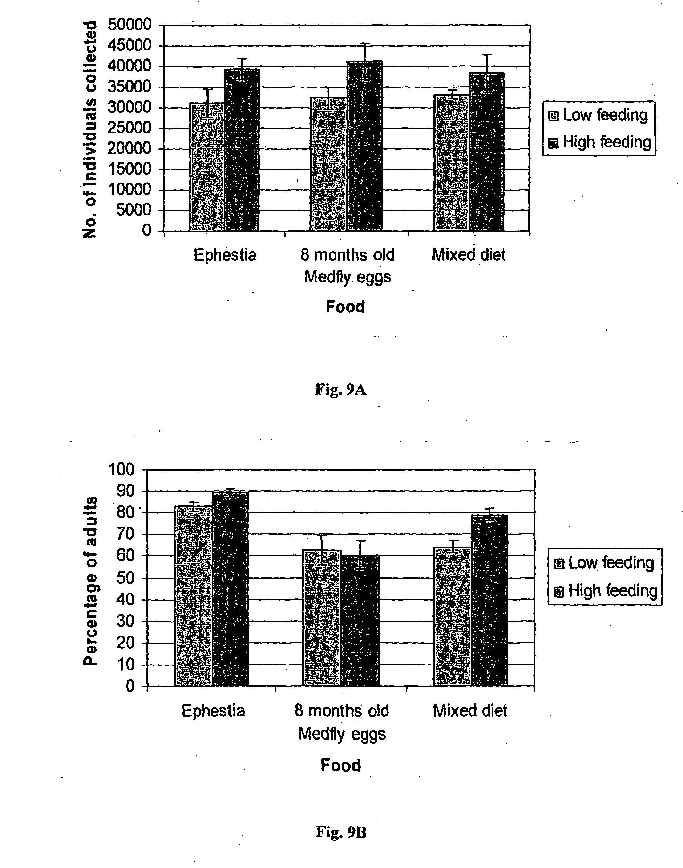

In this example, the result of long-term storage on the PIE was assessed in a bifactorial experiment. Fresh *E. kuhniella* eggs were compared in high and low amounts with medfly PIE that had been stored for 8 months. In addition to the *E. kuhniella* and medfly eggs treatment, a treatment was added in which a mixture of the two diets in a ratio of 30% *E. kuhniella* and 70% medfly eggs was offered. In each treatment approximately 38000 *O. laevigatus* nymphs were fed and each treatment was replicated 5 times. At the end of the experiment the following parameter were assessed; the number of bugs, % of adults, and % mortality. Figure 9A shows that the number of bugs collected was significantly less when fed a low amount of food but that there was no significant difference between the different diets themselves. Thus long-term storage of the PIE for 8 months does not alter its effectiveness as an insect diet for *O. laevigatus.* Moreover, the novel PIE can be mixed in or incorporated with known insect diets to provide an effective diet for BCA's such as *O. laevigatus.*

Mortality rates were negligible in all treatments. Figure 9B illustrates that the percentage of adults was lower for the medfly PIE diet compared with the *E. kuhniella*,diet and intermediate for the mixed diet.

### EXAMPLE 5

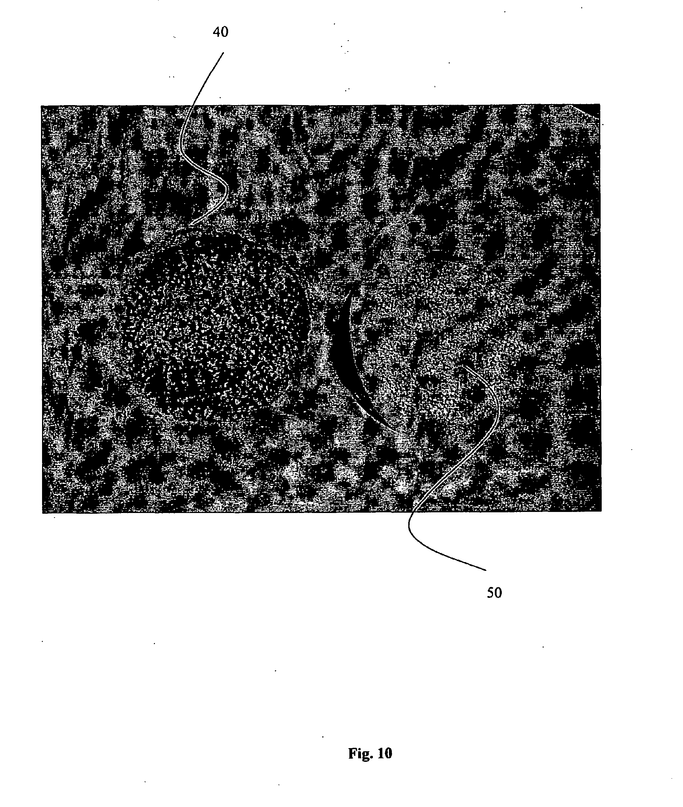

In the standard mass production of *E. kuehniella* eggs, the final product is contaminated with residues of moth adult's scales. Removal of the scales from the eggs may yield a cleaner product and a higher quality food source for predatory arthropods. In an attempt to clean *E. kuehniella* eggs from the scales, eggs were washed with tap water. The moth's scales easily dropped from the eggs and while the eggs sank in the water the scales floated and could easily be removed from the surface of the water. In order to dehydrate the washed *E. kuehniella* eggs, the method of processing Medfly eggs to PIE was implemented, including: water extraction, desiccation by mixing with rice, sifting from the rice, powdering with anti-caking, bagging and quick freezing. Reference is now made to Figure 10 in the drawings, a pictorial illustration of the visual differences between *E. kuehniella* eggs before processing **40** and after undergoing the PIE processing system **50.**

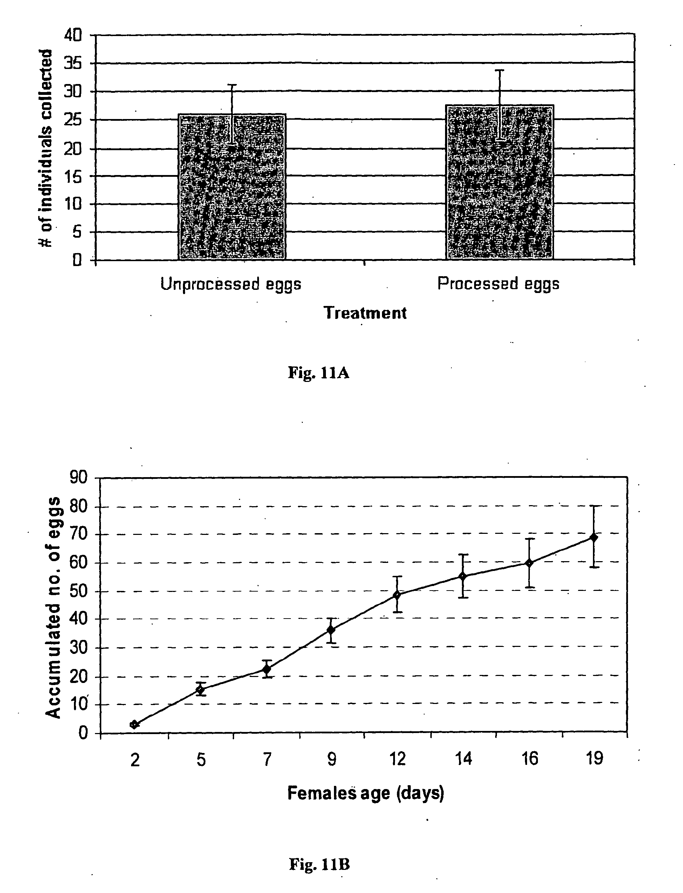

In order to examine the suitability of processed *E. kuehniella* eggs as a food source for predatory bugs, *O. laevigatus* eggs were distributed into 10 small containers. In 5 containers (=replicates) the hatched Orius nymphs were fed with processed *E. kuehniella* eggs and in the other 5 containers nymphs were fed with unprocessed *E. kuehniella* eggs (control). Figure 11 A graphically illustrates that in both treatments there was good development and a similar number of adults was obtained. Figure 11B graphically illustrates that females *O. laevigatus* fed as nymphs with processed *E. kuehniella* eggs, performed a normal oviposition curve in a fecundity test.

### EXAMPLE 6

In this example the Mirid bug *(N. tenius)* was raised on medfly PIE and compared to Mirid bugs raised on *E. kuhniella* eggs. Couples of *N. tenuis* were placed separately in a ventilated arena on sweet pepper leaf disc on agar (N=5) and were provided with medfly PIE or *E. kuhniella* eggs. In both treatments the same fecundity was recorded (ca. 60 eggs per female) with no significant difference. *N. tenuis* in a mass rearing system were successfully fed with a range of diets, including medfly PIE, *E. kuhniella* eggs, as well as a "mix" of medfly PIE with *E. kuhniella* eggs at a ratio of 1:1. The fecundity, fertility, and survival of the *N. tenuis* were found to be as good with medfly PIE and the mixed diet as with rearing on *E. kühniella* eggs only.

### EXAMPLE 7

Adult survival of the ladybeetle (*C. montrouszieri*) on the medfly PIE diet was measured. Couples of *C. montrouzieri* were placed in a ventilated arena (N=12) with medfly PIE, using citrus mealybug eggs and larvae as a control diet. Both treatments showed a survival rate of over 85% after one month.

It is understood that the foregoing detailed description is given merely by way of illustration and that modifications and variations may be made therein without departing from the scope of the invention.

## Claims

1. A system of rearing insect biological control agents (BCAs) which comprises nutrients provided to said BCAs, wherein the nutrients comprise Processed Insect Eggs (PIE), said insect eggs are derived from Tephritid fly species

2. The system according to claim 1, wherein at least one of the following holds true:
a. said insect eggs are derived from medfly eggs by-product of medfly mass production for use in sterile insect technique programs;
b. said insect eggs are derived from medfly or other Tephritid fly species particularly the olive fly;
c. said nutrients are provided by PIE in combination with insect BCA nutrients such as *Artemia* eggs and *Ephestia kuhniella* eggs;
d. said nutrients are provided by PIE in combination with other nutrients or said other nutrients are provided by eggs from at least one other different species that have undergone the PIE processing system or a mixture of PIE and at least one other insect diet supplied to BCA, wherein the at least one other insect diet comprises between about 5% and about 50% of the PIE, further wherein said PIE is derived from Tephritid species.

3. The system according to claim 1, useful for providing nutrients to insect BCA's, wherein said BCA's are selected from the group consisting of the order Hemiptera, the family Miridae, the genus *Nesidiocoris* comprising bugs of the species *Nesidiocoris tenuis,* the family Anthocoridae, the genus *Orius,* comprising minute pirate bugs *Orius laevigatus, Orius insidiosus, Orius* spp., the family Lygaeidae, the genus *Geocoris* comprising big-eyed bugs, *Geocoris spp.,* the order Coleoptera, the family Coccinellidae, the genus *Cryptolaemus,* comprising the mealybug lady beetle *Cryptolaemus montrouzierï,* the order Neuroptera, the family Chrysopidae, the genus *Chrysoperla* comprising the green lacewing, *Chrysoperla* spp., predatory mites from the order Mesostigmata, non- insect BCAs of the Arachnida class, from the family Phytoseiidae. genus *Amblyseius;* comprising the predatory mites; *Amblyseius swirskii* and *Amblyseius* spp., the genus *Phytoseiulus* comprising the predatory mites; *Phytoseiulus persimilis, Phytoseiulus longipes* and *Phytoseiulus* spp., the genus *Euseius* comprising the predatory mites *Euseius scutalis* and *Euseius* spp., or any combination thereof.

4. The system according to claim 1 wherein said PIE is derived from Tephritid species and chemical breakdown of said PIE within the gut of said BCA is significantly different to chemical breakdown of a BCA fed on another type of BCA diet.

5. The system according to claim 1 comprising a factitious diet composition comprising PIE; said PIE having been dewatered, mixed with anti-caking agent, placed in a quick freeze and stored until use under appropriate conditions of below 0°C, wherein said PIE provides nutrients for another different biological control agent further wherein said PIE are derived from an egg supply comprising egg by-products of insect control programs, for example sterile insect technique in medfly, further wherein said PIE is derived from Tephritid species.

6. A method of dewatering insect eggs useful for providing a factitious insect diet comprising the steps of;
a) collecting and storing said eggs under aqueous conditions,
b) determining the volume of eggs collected,
c) separating out a solid phase containing the insect eggs,
d) combining said separated insect eggs with a granular desiccant, and
e) mixing said desiccant and said eggs;
wherein an intermediate product is provided containing said insect eggs in a non-sticky, non-aggregated formulation, further wherein said insect eggs are derived from Tephritid species.

7. The method according to claim 6 of providing an intermediate product of insect eggs, comprising the steps of;
a) storing said collected eggs under aqueous conditions in a refrigerator **10,**
b) determining the volume of eggs by transferring said eggs to a measuring container and removing excess water **12,**
c) dewatering by centrifugation 14 sufficient for separating out a solid phase containing the insect eggs from the aqueous phase,
d) combining separated insect eggs with a granular desiccant, for example fresh rice, at a rate of approximately 2.5 times the known volume of insect eggs **16,**
e) mixing said desiccant with the said insect eggs in a mechanical mixer, with additional manual force for between about 15 to 20 minutes **18,**
wherein an intermediate product is provided containing said insect eggs in a non-sticky, non-aggregated formulation, further wherein said insect eggs are derived from Tephritid species.

8. The method of claim 6 further comprising steps of separating desiccated insect eggs from an intermediate product, by
a) placing said intermediate product in a filtration machine **20,**
b) collecting the granular desiccant in one container **22,**
c) collecting the dewatered insect eggs in another different container **24,** wherein dewatered insect eggs are provided for the production of a factitious insect diet, further wherein said insect eggs are derived from Tephritid species.

9. The method of claim 6 further comprising steps of preparing PIE from said desiccated insect eggs by a) determining the weight of desiccated insect eggs, b) coating said insect eggs with an anti-caking agent, c) mixing the anti-caking agent with said insect eggs to provide a uniform mixture, wherein said mixture is useful for providing a factitious insect diet for insect BCAs, further wherein said PIE is derived from Tephritid species.

10. The method of claim 8 comprising steps of preparing PIE comprising the steps of;
a) weighing said desiccated insect eggs **26,**
b) coating said insect eggs with an anti-caking agent, more particularly rice flour, at a rate of between about 5-8 % of the desiccated eggs' weight **26,**
c) mixing the anti-caking agent with the desiccated insect eggs, to provide a uniform mixture **28,**
wherein said mixture is useful for providing a factitious insect diet for insect BCAs **30,** further wherein said PIE is derived from Tephritid species.

11. The method of claim 6 comprising steps of
a) bagging said PIE into sachets **32,**
b) welding said sachets to seal contents **32,**
c) quick freezing said sachets and contents in a Individually Quick Frozen (IQF) freezer **34,**
d) transferring said sachets to a standard freezer **36,**
wherein the packaged and frozen PIE provided maintains its nutritional attributes and freshness for a period of not less than one year **38,** further wherein said PIE is derived from Tephritid species.

12. The method of preparing and storing PIE, according to claim 6 wherein in any of its preparation, desiccation, mixing and storage stages, said preparation is carried out in a dehumidified environment of relative humidity between about 20% to about 40%.

13. The method according to claim 11, wherein said sachet comprises a high oxygen barrier film useful for maintaining the nutritional attributes and freshness of the PIE for a period of not less than one year.

14. The method according to claim 11, wherein said IQF is useful for maintaining the nutritional attributes and freshness of the PIE for a period of not less than one year further said PIE is suitable for distribution as an insect diet for commercially reared BCAs.

15. A covered or packaged factitious insect diet in covered or packaged form, comprising insect eggs and an anti-caking agent in a sachet, wherein said covered or packaged insect diet is effective for the commercial rearing of a BCA, further wherein said insect eggs are derived from Tephritid species.

## Patentansprüche

1. System zur Aufzucht von Insekten, die als biologische Schädlingsbekämpfungsmittel (Biological Control Agents, BCAs) genutzt werden, welches System Nährstoffe umfasst, die besagten BCAs zur Verfügung gestellt werden, wobei die Nährstoffe verarbeitete Insekteneier (Processed Insect Eggs, PIE) umfassen, wobei diese Insekteneier von Tephritidae-Fliegenspezies stammen.

2. System nach Anspruch 1, wobei mindestens eines der folgenden gilt:
a. die Insekteneier stammen vom Nebenprodukt von Mittelmeer-Fruchtfliegeneiern aus der Massenproduktion von Mittelmeer-Fruchtfliegen zur Anwendung in Sterile-Insekten-Technik-Programmen;
b. die Insekteneier stammen von Mittelmeer-Fruchtfliegen oder anderen Tephritidae-Fliegenspezies, spezieller der Olivenfliege;
c. die Nährstoffe werden durch verarbeitete Insekteneier (PIE) in Kombination mit für Insekten, die als biologische Schädlingsbekämpfungsmittel (BCA) genutzt werden, bestimmten Nährstoffen, wie etwa *Artemia*-Eiern und *Ephestia kuhniella*-Eiern, bereitgestellt;
d. die Nährstoffe werden durch verarbeitete Insekteneier (PIE) in Kombination mit anderen Nährstoffen bereitgestellt, oder besagte andere Nährstoffe werden durch Eier mindestens einer anderen, verschiedenen Spezies, die dem PIE-Verarbeitungssystem unterzogen wurden, oder eine Mischung von verarbeiteten Insekteneiern (PIE) und mindestens einer anderen, biologischen Schädlingsbekämpfungsmitteln (BCAs) zugeführten Insektennahrung bereitgestellt, wobei die mindestens eine andere Insektennahrung zwischen etwa 5% und etwa 50% der verarbeiteten Insekteneier (PIE) umfasst, wobei diese verarbeiteten Insekteneier (PIE) weiter von Tephritidae-Spezies stammen.

3. System nach Anspruch 1, welches nützlich ist, um Nährstoffe für Insekten, die als biologische Schädlingsbekämpfungsmittel (BCAs) genutzt werden, bereitzustellen, wobei diese BCAs aus der Gruppe ausgewählt sind, bestehend aus der Ordnung Hemiptera, der Familie Miridae, der Gattung *Nesidiocoris,* umfassend Käfer der Spezies *Nesidiocoris tenuis,* der Familie Anthocoridae, der Gattung *Orius,* umfassend die Raubwanzen *Orius laevigatus, Orius insidiosus, Orius* spp., der Familie Lygaeidae, der Gattung *Geocoris,* umfassend sogenannte "Big-eyed bugs", *Geocoris spp.,* der Ordnung Coleoptera, der Familie Coccinellidae, der Gattung *Cryptolaemus,* umfassend den Australischen Marienkäfer *Cryptolaemus montrouzieri,* der Ordnung Neuroptera, der Familie Chrysopidae, der Gattung *Chrysoperla,* umfassend die Grüne Florfliege, *Chrysoperla* spp., Raubmilben der Ordnung Mesostigmata, Nicht-Insekten-BCAs der Klasse Arachnida, aus der Familie Phytoseüdae, Gattung *Amblyseus;* umfassend die Raubmilben; *Amblyseus swirskii* und *Amblyseus* spp., der Gattung *Phytoseiulus,* umfassend die Raubmilben; *Phytoseiulus persimilis, Phitoseiulus longipes* und *Phytoseiulus* spp., der Gattung *Euseius,* umfassend die Raubmilben *Euseius sculatis* und *Euseius* spp., oder gleich welche Kombination davon.

4. System nach Anspruch 1, wobei besagte verarbeiteten Insekteneier (PIE) von Tephritidae-Spezies stammen und die chemische Zerlegung besagter verarbeiteter Insekteneier (PIE) im Darm besagter biologischer Schädlingsbekämpfungsmittel (BCA) signifikant verschieden von der chemischen Zerlegung eines BCAs ist, das mit einer anderen Art von BCA-Nahrung ernährt wurde.

5. System nach Anspruch 1, umfassend eine künstliche Nahrungszusammensetzung, die verarbeitete Insekteneier (PIE) umfasst; wobei besagte verarbeiteten Insekteneier (PIE) entwässert, mit Fließhilfsstoffen vermischt, in einer Schock-Tiefkühlvorrichtung platziert und unter geeigneten Bedingungen von unter 0°C gelagert wurden; wobei besagte verarbeitete Insekteneier (PIE) Nährstoffe für ein anderes, verschiedenes biologisches Schädlingsbekämpfungsmittel bereitstellen, wobei diese verarbeiteten Insekteneier (PIE) aus einer Eierzufuhr stammen, die Eier-Nebenprodukte von Insektenbekämpfungsprogrammen, beispielweise Sterile-Insekten-Technik bei der Mittelmeer-Fruchtfliege, umfasst, wobei die verarbeiteten Insekteneier (PIE) weiter von Tephritidae-Spezies stammen.

6. Verfahren zum Entwässern von Insekteneiern, das nützlich zur Bereitstellung einer künstlichen Insektennahrung ist, umfassend die folgenden Schritte:
a) Sammeln und Lagern der Eier unter wässrigen Bedingungen;
b) Ermitteln des Volumens der gesammelten Eier;
c) Abscheiden einer Feststoffphase, die die Insekteneier enthält;
d) Kombinieren der abgeschiedenen Insekteneier mit einem Trocknungsmittel
in Granulatform, und
e) Vermischen des Trocknungsmittels mit den Eiern;
wobei ein Zwischenprodukt bereitgestellt wird, das die Insekteneier in einer nicht klebrigen, nicht-aggregierten Rezeptur enthält, wobei die Insekteneier weiter von Tephretidae-Spezies stammen.

7. Verfahren nach Anspruch 6 der Bereitstellung eines Zwischenprodukts aus Insekteneiern, umfassend die folgenden Schritte:
a) Lagern der gesammelten Eier unter wässrigen Bedingungen in einem Kühlschrank **10;**
b) Ermitteln des Volumens der Eier durch Übertragen der Eier in einen Messbehälter und Entfernen überschüssigen Wassers **12;**
c) Entwässern durch Zentrifugieren **14,** das ausreicht, um eine Feststoffphase, die die Insekteneier aus der wässrigen Phase enthält, abzuscheiden;
d) Kombinieren abgeschiedener Insekteneier mit einem Trocknungsmittel in Granulatform, beispielsweise frischem Reis, mit einem Verhältnis von annähernd 2,5 Mal dem bekannten Volumen von Insekteneiern **16,**
e) Mischen des Trocknungsmittels mit besagten Insekteneiern in einem mechanischen Mischer mit zusätzlicher manueller Kraft für einen Zeitraum von zwischen etwa 15 bis 20 Minuten **18,**
wobei ein Zwischenprodukt bereitgestellt wird, das die Insekteneier in einer nicht klebrigen, nicht-aggregierten Rezeptur enthält, wobei die Insekteneier weiter von Tephritidae-Spezies stammen.

8. Verfahren nach Anspruch 6, weiter Schritte des Abscheidens getrockneter Insekteneier von einem Zwischenprodukt umfassend, durch
a) Platzieren des Zwischenprodukts in einer Filtriermaschine **20,**
b) Sammeln des Trocknungsmittels in Granulatform in einem Behälter **22;**
c) Sammeln der entwässerten Insekteneier in einem anderen, verschiedenen Behälter **24,** wobei entwässerte Insekteneier für die Produktion einer künstlichen Insektennahrung bereitgestellt werden, wobei die Insekteneier weiter von Tephritidae-Spezies stammen.

9. Verfahren nach Anspruch 6, umfassend Schritte des Herstellens verarbeiteter Insekteneier (PIE) aus diesen getrockneten Insekteneiern durch a) Ermitteln des Gewichts getrockneter Insekteneier, b) Überziehen der Insekteneier mit einem Fließhilfsstoff, c) Mischen des Fließhilfsstoffs mit den Insekteneiern, um eine gleichförmige Mischung bereitzustellen, wobei diese Mischung nützlich zur Bereitstellung einer künstlichen Insektennahrung für Insekten, die als biologische Schädlingsbekämpfungsmittel (BCAs) genutzt werden, ist, wobei die verarbeiteten Insekteneier (PIE) weiter von Tephritidae-Spezies stammen.

10. Verfahren nach Anspruch 8, umfassend Schritte der Herstellung verarbeiteter Insekteneier (PIE), umfassend die folgenden Schritte:
a) Wiegen der getrockneten Insekteneier **26,**
b) Überziehen der Insekteneier mit einem Fließhilfsstoff, spezieller Reismehl, mit einem Verhältnis von etwa 5-8% des Gewichts der getrockneten Eier **26,**
c) Mischen des Fließhilfsstoffs mit den getrockneten Insekteneiern, um eine gleichförmige Mischung bereitzustellen **28,**
wobei diese Mischung nützlich zur Bereitstellung einer künstlichen Insektennahrung 30 für Insekten, die als biologische Schädlingsbekämpfungsmittel (BCAs) genutzt werden, ist, wobei die verarbeiteten Insekteneier (PIE) weiter von Tephritidae-Spezies stammen.

11. Verfahren nach Anspruch 6, umfassend die folgenden Schritte:
a) Abfüllen der verarbeiteten Insekteneier (PIE) in Beutel **32,**
b) Verschweißen der Beutel, um den Inhalt zu versiegeln **32,**
c) Schock-Tiefgefrieren der Beutel und des Inhalts in einer Schock-Tiefgefriervorrichtung zum einzeln Tiefgefrieren (IQF) **34,**
d) Übertragen der Beutel in eine Standard-Gefriervorrichtung **36,**
wobei die verpackten und eingefrorenen verarbeiteten Insekteneier (PIE) ihre Nahrungseigenschaften und Frische für einen Zeitraum von nicht weniger als einem Jahr beibehalten 38, wobei die verarbeiteten Insekteneier (PIE) weiter von Tephritidae-Spezies stammen.

12. Verfahren zur Herstellung und Lagerung verarbeiteter Insekteneier (PIE) nach Anspruch 6, wobei diese Herstellung in gleich welcher ihrer Herstellungs-, Trocknungs-, Misch- und Lagerungsstufen in einer entfeuchteten Umgebung mit einer relativen Feuchtigkeit zwischen etwa 20% bis etwa 40% durchgeführt wird.

13. Verfahren nach Anspruch 11, wobei der Beutel eine Folie mit hoher Sauerstoffbarriere umfasst, die nützlich zur Aufrechterhaltung der Nahrungseigenschaften und Frische der verarbeiteten Insekteneier (PIE) für einen Zeitraum von nicht weniger als einem Jahr ist.

14. Verfahren nach Anspruch 11, wobei das einzeln Tiefgefrieren (IQF) nützlich zur Aufrechterhaltung der Nahrungseigenschaften und Frische der verarbeiteten Insekteneier (PIE) für einen Zeitraum von nicht weniger als einem Jahr ist, wobei die verarbeiteten Insekteneier (PIE) weiter geeignet zum Vertrieb als Insektennahrung für kommerziell aufgezogene biologische Schädlingsbekämpfungsmittel (BCAs) sind.

15. Abgedeckte oder verpackte künstliche Insektennahrung in abgedeckter oder verpackter Form, umfassend Insekteneier und einen Fließhilfsstoff in einem Beutel, wobei die abgedeckte oder verpackte Insektennahrung effizient zur kommerziellen Aufzucht eines biologischen Schädlingsbekämpfungsmittels (BCAs) ist, wobei die verarbeiteten Insekteneier (PIE) weiter von Tephritidae-Spezies stammen.

## Revendications

1. Système pouréleverdes agents de controle biologique (BCA) qui sont des insectes, qui comprend des substances nutritives alimentées auxdits BCA, les substances nutritives comprenant des oeufs d'insectes traités (PIE), lesdits oeufs d'insectes dérivant de l'espèce de mouche Tephritidae.

2. Système selon la revendication 1, dans lequel au moins un des éléments repris ci-après est d'application :
a. le sd its oeufs d'insectes dérivent d'un sous-produit d'oeufs de la mouche méditerranéenne d'une production en masse de mouche méditerranéenne à utiliser dans des programmes de techniquesd'élevage de mâles stériles;
b. lesdits oeufs d'insectes dérivent de la mouche méditerranéenne ou d'une autre espèce de mouche Tephiritidae, en particulier de la mouche des oliviers;
c. lesdites substances nutritives sont fornies par les PIE e n combinaison avec des substances nutritives de BCA qui sont des insectes, telles que des oeufs de *Artemia* et des oeufs de *Ephestia kuhniella* ;
d. lesdites substances nutritives sont fournies par les PIE e n combinaison avec d'autres substances nutritives ou bien les dites u autres substances nutritives sont fournies par des oeufs provenant d'au moins une autre espèce différente quia été soumise au système de traitement PIE ou avec un mélange de PIE et d'au moins un régime d'insecte alimenté aux BCA, ledit au moins un autre régime d'insecte comprenant entre environ 5 % et environ 50 % des PIE, et dans lequel lesdits PIE dérivent de l'espèce Tephritidae.

3. Système selon la revendication 1, utile pour procurer des substances nutritives à des BCA qui sont des insectes, lesdits BCA étant choisis parmi le groupe constitué par l'ordre Hemiptem, la famille Miridae, le genre *Nesidiocoris* comprenant des punaises de l'espèce *Nesidiocoris tenuis,* la famille Anthocoridae, le genre Orius, comprenant le punaises s pirates *Orius laevigatus, Orius insidiosus, Orius* spp., la famille Lygaeidae, le genre *Geocoris* comprenant des punaises à gros yeux, *Geocoris* spp., l'ordre Coleoptera, la famille Coccinellidae, le genre *Cryptolaemus,* comprenant la cochenille *Cryptolaemus montrouzierï*, l'ordre Neuroptera, la famille Chrysopidae, le genre *Chrysoperla* comprenant le chrysope, *Chrysoperla* spp., des acariens prédateurs de l'ordre Mesostigmata, des BCA non-insectes de la classe Arachnida, de la famille Phytoseiidae du genre Amblyseius, comprenant les acariens prédateurs *Ambryseius swirskii* et *Ambryseius* spp., le genre Phytoseiulus comprenant les acariens prédateurs *Phytoseiulus persimilis, Phytoseiulus longipe*s et *Phytoseiulus* spp., le genre Euseius comprenant les acariens prédateurs *Euseius scutalis* et *Euseius* spp., o u l'une quelconque d e le urs combinaisons.

4. Système selon la revendication 1, dans lequel lesdits PIE dérivent de l'espèce Tephritidae et la décomposition chimique desdits PIE dans l'intestin desdits BCA est significativement différente de la décomposition chimique d'un BCA alimenté conformément à un autre type de régime de BCA.

5. Système selon la revendication 1, comprenant une composition de régime factice comprenant de PIE, lesdits PIE ayant été déshydratés, mélangés avec un agent anti-agglutinant, placés dans un suigélateuret entre posés jusqu'à l'emploi dans des conditions appropriées inférieures à 0 °C, le sd its PIE procurant d e substances nutritives pour un autre agent de contrôle biologique différent; en outre dans lequel lesdits PIE dérivent d'une alimentation d'oeufs comprenant des sous-produits d'oeufs de programmes de contrôle d'insectes, parexemple de la technique d'élevage de mâles stériles dans la mouche méditerranéenne ; en outre dans lequel lesdits PIE dérivent de l'espèce Tephritidae.

6. Procédé de déshydratation d'oeufs d'insectes utiles pour procurer un régime d'insecte factice comprenant les étapes consistant à :
a) recolter et entreposer lesdits oeufs dans des conditions aqueuses;
b) déterminer le volume des oeufs récoltés ;
c) séparer une phase solide contenant les oeufs d'insectes;
d) combiner lesdits oeufs d'insectes sépares avec un dessiccateur granuleux ; et
e) mélanger ledit dessiccateur et lesdits oeufs ;
dans lequel on procure un produit intermédiaire contenant lesdits oeufs d'insectes dans une formulation non collante, non agglomérée, en outre dans lequel lesdits oeufs d'insectes dérivent de l'espèce Tëphritidae.

7. Procédé selon la revendication 6, pour l'obtention d'un produit intermédiaire d'oeufs d'insectes, comprenant les étapes consistant à :
a) entreposer lesdits oeufs recoltés dans des conditions aqueuses dans un refrigérateur 10,
b) déterminer le volume des veufs en transférant lesdits oeufs à un récipient de mesure et éliminer l'eau en excès 12,
c) déshydrater par centrifugation 14 suffisante pour séparer de la phase aqueuse une phase solide c o nte na nt le s oeufs d'insectes,
d) combiner les veufs d'insectes séparés avec un dessiccateur granuleux par exemple du riz frais en une quantité correspondant approximativement à 2,5 fois le volume connu des oeufs d'insectes 16,
e) mélanger ledit dessiccateur avec lesdits oeufs d'insectes dans un mélangeur mécanique auquel on ajoute une force manuelle pendant environ 15 à 20 minute s 18,
dans lequel on obtient un produit intermédiaire contenant le sd its oeufs d'insectes dans une formulation non collante, non agglomérée, en outre dans lequel lesdits oeufs d'insectes dérivent de l'espèce Tephritidae.

8. Procédé selon la revendication 6, comprenant en outre les étape consistant à séparer d'un produit intermédaire les oeufs d'insectes déshydratés s
a) en plaçant ledit produit intermédiaire dans un dispositif filtra tio n 20,
b) en récoltant le dessiccateur granuleux dans un récipient 22,
c) en récoltant les oeufs d'insectes déshydratés dans un autre récipient différent 24, dans lequelles oeufs d'insectes s déshydratés s sont fournis pour la production d'un régime d'insecte factice, en outre dans lequel lesdits oeufs d'insectes dérivent de l'espèce Tephritidae.

9. Procédé selon la revendication 6, comprenant en outre les étapes de préparation des PIE à partirdesdits oeufs d'insectes desséchés para) détermination du poids des oeufs d'insectes desséchés, b) enrobage desdits oeufs d'insectes avec un agent anti-agglutinant, c) mélange de l'agent anti-agglutinant avec lesdits oeufs d'insectes pour obtenir un mélange uniforme, dans lequel ledit mélange est utile pour procurer un régime d'insecte factice pour des BCA qui sont des insectes, en outre dans lequelles dits PIE dérivent de l'espèce Tephritidae.

10. Procédé selon la revendication 8, comprenant les étapes de préparation des PIE comprenant les étapes consistant à :
a) peser lesdits oeufs d'insectes desséchés 26,
b) enrober les dits oeufs d'insectes avec un agent anti-agglutinant, plus particulièrement de la farine de riz, à un taux entre environ 5 et 8 % du poids des s oeufs desséchés 26,
c) mélanger l'agent anti-agglutinant avec les tufs d'insectes desséchés, pour obtenir un mélange uniforme 28,
dans lequel ledit mélange est utile pour procurer un régime d'insecte factice pour des BCA qui sont des insectes 30, en outre dans lequel les dits PIE dérivent de l'espèce Tephritidae.

11. Procédé selon la revendication 6, comprenant les étapes consistant à :
a) ensacher les dits PIE dans des sachets 32,
b) souder les dits sachets pour sceller le contenu 32,
c) surgeler lesdits sachets et leur contenu dans un surgélateur pour surgélation séparée (IQF) 34,
d) transférer les dits sa c he ts à un surgélateur standard 36,
dans lequel les PIE conditionnés et congelés procurent le ma intie n d e leurs attributs nutritifs et leur fraîcheur pendant un laps de temps qui n'est pas inférieur à une année 38, en outre dans lequel lesdits PIE dérivent de l'espèce Tephritidae.

12. Procédé de préparation et d'entreposage de PIE, selon la revendication 6, danslequelau counde l'une quelconque des étapes de leur préparation, de leur dessèchement, de leur mélange et de leur entreposage, ladite préparation est mise en oeuvre dans un environnement déshumidifié dont l'humidité relative se situe entre environ 20 % et environ 40 %.

13. Procédé selon la revendication 11, dans lequel ledit sachet comprend un film procurant une barrière élevée à l'oxygène, utiles pour maintenir les attributs nutritifs e la fraîcheur des PIE pendant un laps de temps qui n'est pas inférieur à une année.

14. Procédé selon la revendication 11, dans lequel ledit IQF est utile p o ur m a inte nir le attributs nutritifs e la fraîcheur de s PIE pendant un laps de temps qui n'est pas inférieur à une année, en outre le sd its PIE sont appropriés pour une distribution à titre de régime d'insectes pour des BCA élevés à l'échelle industrielle.

15. Régime d'insecte factice recouvert ou conditionné, sous une forme recouverte ou conditionnée comprenant des oeufs d'insectes et un agent anti-agglutinant dans un sachet, dans lequel ledit régime d'insectes recouverts ou conditionnés est efficaces pour l'élevage de BCA à l'échelle industrielle, en outre dans lequel lesdits oeufs d'insectes dérivent de l'espèce Tephritidae.
